# EUROPEAN PATENT APPLICATION

(11) **EP 4 201 343 A1**
(43) Date of publication of application: **28.06.2023**
(21) Application number: 21383220.7
(22) Date of filing: 24.12.2021
(51) Int. Cl.: A61B 10/00

(54) **A SALIVA MONITORING ORAL DEVICE FOR COLLECTING AND MONITORING A SALIVA SAMPLE**

(71) Applicant: Happy Innova SL, 17003 Girona (ES); Agencia Estatal Consejo de Investigaciones Científicas (CSIC), 28010 Madrid (ES)
(72) Inventor: BALLART CERDÁN, Diana, 17003 Girona (ES); GARCIA PUIG, Roger, 08221 Terrassa (ES); VALETA ARCARONS, Albert, 08037 Barcelona (ES); MUÑOZ BERBEL, Francesc Xavier, 08193 Bellaterra (ES); GIMÉNEZ GÓMEZ, Pablo Antonio, 30319 Cartagena (ES)
(74) Representative: Curell Suñol S.L.P.

(57) **Abstract**

The invention refers to a saliva monitoring oral device (1) for collecting and monitoring a saliva sample of a user. The device comprises a head (2) configured to be received in the user's mouth, and a longitudinal handhold means (4) with a first and a second ends (6, 8). The head (2) and the handhold means (4) are both hollow and fluidically connected to one another at the first end (6) of the hollow handhold means (4) to define a saliva collection chamber (10). The hollow head (2) is rigid, insoluble in saliva and comprises a plurality of inlet through holes (12). The plurality of inlet through holes (12) are fluidically connected to the saliva collection chamber (10) for allowing, in use, saliva from the user's mouth to enter the saliva collection chamber (10). The device (1) further comprises saliva sensing means (16) comprising a reactive agent for characterising the saliva sample.

## Description

### Field of the invention

The invention relates to a saliva monitoring oral device for collecting and monitoring a saliva sample of a user comprising: a head configured to be received in the user's mouth, and longitudinal handhold means with a first and a second ends.

### State of the art

Saliva monitoring oral devices are known in the art. This kind of devices is often intended to assess saliva either for health purposes or for detection of other analytes such as drugs, alcohol or similar substances.

Document US 2002/127143 A1 discloses a biosensor electrical toothbrush having a brush head with a test channel and a renewable biosensor system within the test channel for performing routine saliva tests. This device does not allow the collection of relevant amounts of saliva for later analyses in a laboratory. Furthermore, the saliva sample can easily be contaminated, leading to misleading results.

Document US 2004/220498 A1 discloses an oral platform, a microchip for making physiological tests and/or delivery of drugs, and a stick connected to the platform to serve as a handle or conduit from the microchip for exterior communication. A candy shell coating on the platform incorporates medicinal agents. The platform has a plurality of fluidic ports conductive for communication of saliva to or oral delivery from the microchip. Again, the quantity of saliva collected is small, not allowing to carry out later tests, in addition to on site tests. Furthermore, the device is not pleasant to use because once the candy has melted with saliva, the user's oral cavity is in direct contact with the microchip.

Document WO 2010/008989 A2 discloses a device for non-invasive analysis of an oral fluid, such as saliva, to determine the presence and the level of a certain saliva component. The apparatus includes a user-friendly, lollipop like construction that is selfcontained and provides the user with a method to obtain a saliva volume by sucking or licking the pop-like head of the apparatus. Again, the saliva sample acquired with this device is reduced and collected from the outside of the lollipop with a sucking device. The sample can be easily contaminated.

### Summary of the invention

It is an object of the invention to propose a saliva monitoring oral device for collecting and monitoring a saliva sample of a user which facilitates collecting larger amounts of saliva in a short period of time, that is easy to use and that at the same time avoids the risk of contamination of the saliva sample with external agents. This purpose is achieved by a saliva monitoring oral device of the type indicated at the beginning, characterised in that said head and said handhold means are both hollow and fluidically connected to one another at said first end of said hollow handhold means to define a saliva collection chamber, said hollow head is rigid, insoluble in saliva and comprises a plurality of inlet through holes, said plurality of inlet through holes being fluidically connected to said saliva collection chamber for allowing, in use, saliva from the user's mouth to enter said saliva collection chamber, said device further comprises saliva sensing means containing a reactive agent for characterising said saliva sample.

The hollow head of the device takes advantage of the new-born sucking reflex of the mammals. Therefore, the invention is preferably applicable to humans, but it could also find application in other mammals of animal origin. Thanks to the rigid hollow head, which is not soluble in saliva, when the device is inserted into the user's mouth, the roof of the mouth is contacted during the sample collecting process. This constant contact causes this suction reflex movement, accompanied by a saliva secretion increase.

On the other hand, the inlet through holes and the hollow head, facilitate that a large amount of the secreted saliva directly flows through the inlet through holes, no matter what the position of the hollow head of the device in the mouth, or the rotation of the device during the sucking reflex. The saliva is then directed towards the inside of the device that is into the saliva collection chamber. Directing the saliva towards the inside of the device is again an important advantage over the prior art devices, since the saliva collected into the saliva collection chamber is, firstly stored and accumulated in the collection chamber and secondly it is further protected from contamination. In prior art devices, most of the saliva cannot be collected into the device and remains in the user's mouth. In other cases, the saliva remains on the surface of the device, exposed to contamination. Therefore, the amount of saliva collected by the device of the invention is increased over the prior art devices and at the same time the risk of contamination of the sample is eliminated.

The device according to the invention has the advantage over other prior art devices that it allows not only to perform screening analyses in situ with small amounts of saliva, but also to perform more accurate analyses in the laboratory. In particular, these larger amounts of saliva are well protected from contamination and can thus be preserved and sent to a laboratory for carrying out more detailed analyses at a later moment of time.

A further advantage of the device according to the invention, is that the lollipop structure of the device makes it very easy to use. This is a very relevant advantage for collection of saliva samples in children or elderly people.

Yet another advantage derived from the simplicity of use, is that the device can be used in an unsupervised manner and without requiring any expert accompanying the user during the saliva sample collection. This makes screening campaigns more simple and secure in the case of detection of analytes related to very contagious diseases.

The invention further includes a number of preferred features that are object of the dependent claims and the utility of which will be highlighted hereinafter in the detailed description of an embodiment of the invention.

In order to increase the precision of the characterization of the saliva sample preferably said saliva sensing means further comprises at least one sensing film and said reactive agent is contained in said at least one sensing film, said at least one sensing film being arranged inside said hollow handhold means, in fluidical communication with said saliva collection chamber, distanced from said hollow head, such that, in use, said at least one sensing film is impregnated by the saliva collected inside said saliva collection chamber. Thanks to the fact that said reactive agent is located in the collecting chamber, but outside the hollow head, and inside the device, the reactive agent is not in direct contact with the user's mouth anymore, and protected from the outside, thus further protecting the saliva sample. The harmfulness of the reactive agent depends not only on the own toxicity thereof, but also in other parameters such as concentration, its solubility with saliva, capacity of penetration in the human body, user's exposure time and others. Therefore, by separating the reactive agent from the user's mouth, the device is even more secure. Now it can be chosen any reactive agent at any concentration, such as to reach the most optimal parameters for the analytical response. In other words, it is no longer required to take into account aspects of toxicity that the other systems do have to consider, such as the physical-chemical characteristics of the compound, its solubility in the mouth through saliva, its capacity to penetrate the human body, its exposure time or the like.

In an embodiment seeking for a simple and reliable characterization said hollow handhold means comprises at least one optical path located between said first and second ends and distanced from said hollow head, and in that said at least one sensing film is arranged to cover said optical path. In this way the device has a reduced cost, thus allowing it to be conceived for one use only.

In an embodiment seeking to collect the saliva more rapidly, said hollow head has an inner volume of at least 2 mL. This size has been found to further facilitate the entrance and collection of saliva inside the hollow head. Furthermore, such a large amount of saliva is especially adequate for later laboratory more precise analyses.

In another embodiment, seeking to further increase the amount of saliva collected, the device comprises saliva absorption means, said saliva absorption means being arranged within said collection chamber, inside said hollow head, to absorb, in use, saliva flowing into said hollow head. The absorption means can be made of a sponge-like material, cellulose, or the like. Thanks to the arrangement of this absorption means within the collection chamber, protected by the rigid hollow head, it is avoided that the saliva entering through the inlet through holes exits the collection chamber through another through hole of the hollow head. But furthermore, the saliva absorption means absorb the saliva from the user's mouth towards the inside of the collection chamber. Again, this maximises the amount of saliva introduced and retained within the collection chamber. Preferably, said absorption means completely fill up the hollow head's inner volume to absorb the saliva as soon as it crosses the inlet holes. It must be noted that in many prior art devices absorption means are intended to come into direct contact with the user's mouth. This has several drawbacks. First this is unpleasant for the user because of the feel of the absorption means. This can even lead to a reduction of the saliva secretion. Furthermore, this unpleasant feeling can also lead to the user squeezing the absorption means, thus draining the absorption means during the collection thus reducing again the amount of collected saliva.

The optical path can be easily achieved by using a transparent material for making the hollow handhold means. However, in a solution seeking for an optimization of the characterising results, that is, that no modifications are caused due to other parts of the device, different to the sensing film, preferably said optical path comprises at least one monitoring through hole on the hollow handhold means. Preferably said monitoring through hole is closer to said second end than to said first end of the hollow handhold means.

In another embodiment, said at least one monitoring through hole extends through the cross section of said hollow handhold means.

In another preferred embodiment, said hollow handhold means further comprises saliva displacement means fluidly associated to said saliva collection chamber to cause saliva collected during the use of said device to be displaced from said hollow head towards said sensing film. Again, this makes the saliva collection easier and with larger quantities.

Also in order to maximise the saliva sample to be analysed said saliva displacement means comprises an inner plunger with a plunger head, said inner plunger being guided within said hollow handhold means, slidably mounted between an inserted position in which said plunger head is adjacent to said hollow head and a remote position in which said plunger head is located near said second end, beyond said sensing means, to the opposite to the side of said first end. Thanks to the depression caused within the saliva collection chamber, when the plunger is moved between the inserted position and the remote position, the saliva sample is drawn to the bottom of the collection chamber, where the sensing means are arranged, thus maximising the impregnation of the sensing film.

Although the depression in the hollow chamber draws large quantities of saliva towards the reactive agent, preferably said hollow handhold means comprises a tubular tip at said first end having a smaller cross section than the cross section of the hollow handhold means and in that said tubular tip reaches the inner volume of said hollow head and is fluidically connected to said saliva absorption means. This configuration has several advantages. First the depression is created directly against the absorption means, thus increasing the amount of saliva harvested. Furthermore, the tip acts as a stopper, avoiding that the depression created draws the absorption means down towards the bottom of the collection chamber and hampering a correct lecture of the sensing film. Preferably said tubular tip is abutting against said saliva absorption means.

Furthermore, although the device enhances the saliva secretion based on mechanical tools, such as the shape of the hollow head, it could be further enhanced chemically, thus even improving the results obtained. To this end, the hollow head further comprises an outer saliva secretion enhancing layer, said saliva secretion enhancing layer being chemically inert, both for the user and for the measurements. It is important that the saliva secretion enhancing layer does not modify the results of the sample. Being chemically inert, its only function is to stimulate the salivary glands to produce more saliva. This layer is preferably soluble with saliva. Also preferred tastes for the saliva enhancing layer are citric flavours such as orange, lemon, strawberry or the like, making for the user a more pleasant experience to obtain the saliva sample, and again facilitating the saliva secretion.

It has been found that when said sensing film is distanced a distance L of at least 20 mm from the outer surface of said hollow head, and preferably at least 30 mm it is guaranteed that the user does not get in contact with the reactive agent, with the aforementioned advantages.

Also, to allow the diagnosis of different diseases or analytes in a fast and cost-effective manner, the saliva sensing means comprises at least two sensing films and each sensing film contains different reactive agents.

Furthermore, in a preferred transduction option said saliva sensing means further comprises: a light emitting source arranged to illuminate said at least one sensing film with a light beam, a light receiving sensor, arranged facing said at least one sensing film to receive said light beam from said at least one sensing film, and processing means functionally associated to said light emitting source and said light receiving sensor to analyse said light beam and to correlate it with the target analyte to be detected. The light receiving sensor can be arranged facing the same side of said at least one sensing film as the light emitting source, thus receiving the light beam reflected on the sensing film, or alternatively it can be arranged facing the opposite side of said at least one sensing film which the light emitting source is facing, thus receiving the light beam crossing the sensing film.

Preferably said absorption means are made of a sterile and clinically compatible absorption material to avoid any interaction with saliva that could affect the results of the characterization.

However, it has been noticed that the more pleasant the feeling of the device in the user's mouth, the more saliva is secreted by the user. Therefore, to further stimulate the saliva secretion, in a preferred embodiment said hollow head has an overall size of at least 15 mm, which size has been found to be the starting size to cause a pleasant stimulation of the user's oral cavity. For human applications it is preferred that the overall size of the hollow head is comprised between 15 and 35 mm.

Preferably said hollow head is lollipop-shaped and has a shape among one of the group consisting of a sphere, a cube, a parallelepiped, a cylinder, any of the previous forms having rounded edges to provide a more pleasant experience to the user.

Alternatively, said hollow head is shaped as the head of a pacifier. This improves the saliva collection in the case of very small children below 3 years old.

Even more preferably said hollow head consists of convex rounded surfaces to improve the massaging experience in the mouth.

In an especially preferred embodiment, said hollow head consists of a revolution body obtained from the rotation of an externally convex curve around a rotation axis, to provide a homogenous massage of the device in the mouth, this leading to a more constant saliva secretion. Preferred generatrix curves in this case are a semi-circle or a semi-ellipse, rotated about a rotation axis it being their corresponding symmetry axis.

It is also especially preferable that the hollow head consist also internally of a revolution body obtained from the rotation of an externally convex curve around a rotation axis. Such curves facilitate the saliva to remain in the collection chamber. Preferred generatrix curves in this case are a semi-circle or a semi-ellipse, rotated about a rotation axis it being their corresponding symmetry axis.

Preferably the wall thickness of the hollow head for optimal production of the part is between 0.5 and 3 mm, and preferably between 1 and 2.5 mm.

Preferably each hole of said plurality of inlet through holes in the hollow head have a size between 0.5 and 10 mm, e.g., if the holes are circular, they have a diameter of 0.5 to 10 mm, and if they are square, they have side sizes among the cited range. This size range is found to provide a good balance between user's comfort and saliva collection possibilities.

Preferably the hollow handhold means can have a geometry such as cylindrical, prismatic, parallelepipedal or the like that is with a cross-section such as circular, triangular, square or other extending longitudinally along a central axis.

Preferably, said longitudinal hollow handhold means is an elongated hollow shaft configured to provide a handhold for the user's hand.

Preferably the hollow handhold means is a hollow shaft with a maximum cross section comprised between 2 and 6 mm and a length between 70 and 100 mm which has found to be the optimum size for the user to hold the device during the sample collection procedure. Furthermore, the hollow handhold means has a wall thickness of 0.5 to 1.5 mm.

When the hollow head is shaped as the head of a pacifier, said handhold means is a short cylindrical hollow body.

Preferably said plurality of inlet through holes is distributed along the whole hollow head surface, thus providing many different possible saliva entry points and leading to a maximisation of saliva collection probability. In order to simplify the production the through holes have parallel axes, thus simplifying the production of the hollow head by injection moulding.

Preferably said plurality of inlet through holes are uniformly distributed along the hollow head surface, to obtain a more homogeneous saliva collection pattern.

Also preferably said inlet through holes have all the same size to collect the saliva sample homogenously along the perimeter of the hollow head.

Especially preferably, said plurality of inlet holes, comprises between 5 and 50 inlet holes. In a preferred embodiment seeking for a more pleasant feeling in the mouth, said plurality of inlet holes comprises between 5 and 30 inlet holes, which balances the inlet hole area, with the excess of holes on the surface of the hollow head. The larger the number of inlet holes, the rougher feeling for the user in the user's mouth.

Likewise, the invention also includes other features of detail illustrated in the detailed description of an embodiment of the invention and in the accompanying figures.

### Brief description of the drawings

Further advantages and features of the invention will become apparent from the following description, in which, without any limiting character, preferred embodiments of the invention are disclosed, with reference to the accompanying drawings in which:
Figure 1, a front view of a first embodiment of the saliva monitoring oral device according to the invention for collecting and monitoring a saliva sample of a user.
Figure 2, a diagrammatic longitudinal section view of the device of Figure 1.
Figure 3, a perspective view of a second embodiment of the saliva monitoring oral device according to the invention for collecting and monitoring a saliva sample of a user.
Figure 4, a front view of the device according to Figure 3.
Figure 5, a side view of the device according to Figure 3.
Figure 6, a perspective longitudinal section view of the device of Figure 3 without saliva displacement means.
Figure 7, a perspective longitudinal section view of the complete device of the second embodiment.
Figure 8, a perspective view of the saliva displacement means of the device of Figure 7.
Figure 9, a perspective view of the device of Figure 3 during the sample analysis.
Figure 10, a partial cross-section view in perspective of the optical path of the device of Figure 3 during the sample analysis.
Figure 11, a partial cross-section view in perspective of a second alternative embodiment of the optical path of the device according to the invention.
Figure 12, a partial cross-section view in perspective of a third alternative embodiment of the optical path of the device according to the invention.
Figure 13, enzyme reactions to determine total cholesterol.
Figure 14, enzyme reactions to determine the HDL cholesterol.
Figure 15, absorbance spectrum for the determination of cholesterol.
Figure 16, calibration line for the determination of cholesterol concentration.
Figure 17, a diagrammatic front view of a third embodiment of the device according to the invention.

### Detailed description of embodiments of the invention

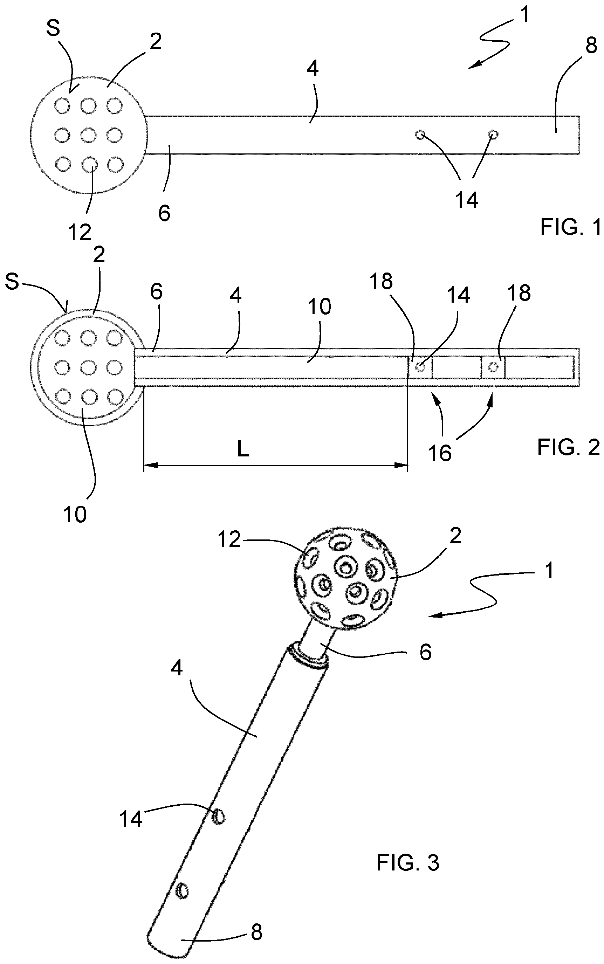

Figures 1 and 2 show a first embodiment of the saliva monitoring oral device 1 according to the invention for collecting and monitoring a saliva sample of a user. This device 1 is intended to monitor the saliva sample collected in an economic and efficient way, either for disease diagnosis or other saliva testing examples, such as drug testing, or the like. It also improves the designs of the prior art thanks to the fact that it takes advantage of the new-born sucking reflex of the mammals to collect larger amounts of saliva . Thanks to the larger amount collected, later tests carried out on the saliva sample are also more reliable. Furthermore, the device according to the invention is easier to use in requires no professional supervision of use.

The saliva monitoring oral device 1 of Figures 1 and 2 comprises as main parts a head 2 configured to be received in the user's mouth and a longitudinal handhold means 4 with a first and a second ends 6, 8. The handhold means 4 of this embodiment is an elongated hollow shaft, having a circular outer cross section.

In this preferred embodiment, these outer parts are made of materials that meet USP Class VI or ISO 10993-1 biocompatibility certifications. This guarantees that the saliva monitoring oral device 1 is biocompatible for up to 30 days in contact with the human body. An example of a material meeting such requirements is ABS Medical Smartfil.

The head 2 and the handhold means 4 are both hollow and fluidically connected to one another at the first end 6 of the hollow handhold means 4. Both parts together define a saliva collection chamber 10, in which in use, the saliva of the user can be collected for later analysis.

The hollow head 2 of the saliva monitoring oral device 1 is rigid and insoluble in saliva. Furthermore, in this embodiment the hollow head 2 is externally spherical having a diameter of at least 15 mm. Internally, the hollow head is also spherical. As it is apparent from these figures, the hollow head 2 comprises a plurality of inlet through holes 12 which are preferably comprised between 5 and 50, and more preferably for simplifying the production of the head, without reducing the saliva inlet, between 5 and 30. This plurality of inlet through holes 12 are fluidically connected to the saliva collection chamber 10. In use, these inlet through holes 12 allow that saliva from the user's mouth enter the saliva collection chamber 10. It is also preferable that the head 2 has an inner volume of at least 2 mL.

The plurality of inlet through holes 12 are distributed along the whole hollow head 2 surface. It is especially preferable that the inlet through holes 12 are evenly distributed along the hollow head surface S and that they have the same size, this not being though essential for the invention.

The device 1 further comprises saliva sensing means 16 containing a reactive agent for characterizing the saliva sample.

As already mentioned, the device 1 according to the invention must enhance the collection of larger amounts of saliva. Also, to gain in reliability of the saliva analysis for the characterization the risk of contamination of the sample with external agents must be eliminated.

The first goal is achieved by the lollipop shape of the device 1 with a hollow head 2 in combination with all the inlet through holes 12 of this first embodiment. Once the device 1 is inserted into the user's mouth, the device 1 is in contact with the palate of the user. This contact with the palate triggers the sucking reflex of the user, thus making the salivary glands increase the saliva secretion. It is then when the saliva easily enters the saliva collection chamber 10 through the inlet through holes 12. But then, once the saliva has entered the chamber 10, it is difficult that it flows outside again, because the collection chamber 10 extends in this case through the hollow handhold means 4. The hollow head 2 does not melt through saliva, such that this mechanical saliva secretion effect remains during the whole saliva collection procedure. The more saliva produced; the more saliva enters the collection chamber 10.

Then the second goal of avoiding contamination of the saliva sample is achieved because the saliva sample is kept, not on the surface S of the device 1 like other prior art devices, but in the collection chamber 10.

In an especially preferable embodiment, since the hollow handhold means 4 is normally inclined downwards when the device 1 is in the user's mouth or held in his hands, the collected saliva flows downwards towards the second end 8 of the hollow handhold means 4. During the saliva flow towards the second end 8 the saliva contacts the sensing means 16, such that the sample is at least partially retained therein for later analysis, and completely protected from the environment.

In this preferred embodiment, the sensing means 16 comprises two sensing films 18 and the reactive agents are contained in these sensing films 18 that are arranged in fluidical communication with the saliva collection chamber 10.

The sensing films 18 have preferably a thickness between 100 µm and 1000 µm. These sensing films 18 contain the reactive agent that enables the determination of the analyte of interest. Suitable reactive agents according to the invention would be, among others, enzymes, redox mediators, surfactants, antibodies, antigens, DNA and RNA oligomers, etc... The sensing films 18 can be attached to the device in different ways, such as pressure-sensitive adhesives, by incorporating some external structure concentric to the collection part or by chemical reaction, among others.

In this preferred embodiment the sensing films 18 are distanced from the hollow head 2. In use, they are impregnated by the saliva collected inside said saliva collection chamber 10. Also, when the sensing film 18 is distanced a distance L of at least 20 mm from the outer surface S of said hollow head 2 the precision of the device 1 can be increased. Since the reactive agents are not in contact with the user's mouth, any reactive agent at any concentration can be chosen. This provides the most optimal parameters for the analytical response with the device and the user's adverse reaction to the reactive agent can be avoided.

The sensing films 18 can be produced using bio-functionalization technology, such as conductive polymers, hydrogels, nanocomposites, silk, etc. to enable "reactive agentfree" biosensors to be obtained.

The sensing films 18 are synthesised under optimal conditions, i.e., the amount of reactive agents, proportion of the reactive agents when they are a mix of several agents, crystallisation conditions, etc., to ensure that they are reproducible, robust, cost-effective and with an adequate analytical response. Under analytical response it is understood a high sensitivity, low detection limit, wide linear response range, high selectivity, high reproducibility, etc. in agreement with concentration range of the analyte of interest in the saliva.

The transduction method used to integrate the sensing means 16, i.e., the method for interpreting the biological recognition reaction and translating it into a quantifiable signal can be of any type. Among other options, the method can be optical, electrochemical, piezoelectric, magnetic or any other form of internal transduction or any non-integrated external equipment that allows the biological recognition reaction taking place in the device 1 to be quantified and interpreted.

However, in a preferred embodiment of the invention, the transduction method is optical. To this end, in the device 1 of Figures 1 and 2, the hollow handhold means 4 has two optical paths 14 located between the first and second ends 6, 8. Furthermore, these two optical paths are distanced from said hollow head 2, near to the second end 8. As it is apparent, the sensing films 18 are arranged to cover the optical paths 14. The optical paths 14 in this case are monitoring through holes 22 at the second end 8 extending through the cross section of the hollow handhold means 4.

For the optical transduction method, the sensing films 18 have a high degree of transparency, optimised to ensure the optical measurement. In the sensing films 18, a chemical mediator with optical properties is added, which undergoes a colour change after a series of cascade reactions occur when the saliva containing the analyte under study comes into contact with the sensing films 18.

The colour change will be proportional to the concentration of the analyte of interest in the saliva sample collected from the user. This allows to quantify the exact concentration of analyte in the user's saliva.

The saliva sensing means 16 comprises a plurality of sensing films 18. In this case, two sensing films 18 are arranged on the optical paths 14. In an embodiment looking for an economical way to diagnose a plurality of diseases each sensing film 18 contains a different reactive agent. This allows for a multiplexed measurement system. It is only necessary to adapt the number of optical paths 14 of the device 1 to the simultaneous number of analytes that is to be determined.

The device 1 according to the invention, can be applied for collecting and monitoring of, among others, metabolic indicators, such as glucose, lactate, pyruvate, succinate, etc. These indicators present in saliva can be measured enzymatically and thus used as biomarkers of a plurality of pathologies, such as celiac disease, cardiovascular diseases, cancer, infectious diseases, mental illnesses or the like. Other analytes as biomarkers(e.g., by means of an immunological assay such as aptamers or any other natural or artificial molecule capable of recognising the compound of interest), drugs of abuse, infectious diseases of viral or bacterial origin can also be detected. The device according to the invention can be applied to the measurement and quantification in saliva of any analyte of a protein, hormone, nucleic acid (DNA, RNA, IncRNA, etc.) or organic compound, among others, both in situ, or later in a laboratory, thanks to the large amount of collectable saliva.

Later it will be further explained how the collected sample can be used for diagnostic purposes.

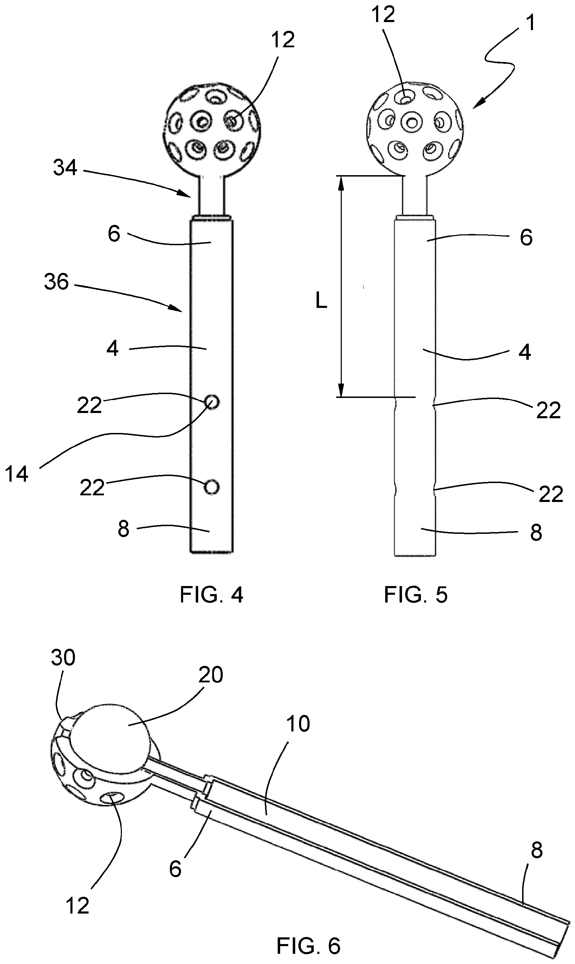

Figures 4 to 10 show a second embodiment of the saliva monitoring oral device 1 according to the invention sharing many common features with the device already described in the previous paragraphs. Therefore, for this second embodiment the common features are not always newly described, and reference is made to the previous paragraphs.

Further to the hollow head 2 with a plurality of inlet through holes 12 and the hollow handhold means 4 being fluidically connected to one another to define a saliva collection chamber 10 and saliva sensing means 16 containing reactive agents for characterising said saliva sample this embodiment further seeks to increase the amount of saliva collected.

The hollow head 4 is again lollipop-shaped, in this case in the shape of a sphere. The overall outer size of the hollow head is configured to be introduced in the user's mouth. The hollow head has an outer size of at least 15 mm. To make an easier production, the wall thickness of the hollow head is of about 0.5 to 3 mm.

The inlet through holes 12 in the hollow head 2 are conically tapering towards the collection chamber 10, having a diameter between 0.5 and 10 mm.

In this preferred embodiment the hollow handhold means 4 is cylindrical. This cylindrical handhold means is between 70 mm and 100 mm long and consists of two zones of different diameters: a first zone 34 closer to the hollow spherical head 2 between 15 mm and 30 mm in length, between 2 mm and 6 mm in external diameter and between 1 mm and 5 mm of internal diameter. Then the hollow handhold means has a second zone 36 which is thicker. This second zone is between 40 mm and 70 mm long, between 4 mm and 10 mm in external diameter and between 3 mm and 9 mm in internal diameter. This area has further two cylindrical holes with a diameter between 1 mm and 5 mm. Its geometry with two zones of different diameters has a dual function: it ensures that the hollow collecting part can be comfortably inserted into the user's mouth, and it also allows the device 1 to be held correctly and comfortably during the sample collection process.

It has been noticed that since saliva is generated mainly in the lower and back part of the mouth by the salivary glands, the amount of saliva collected can be increased if the device 1 further comprises saliva absorption means 20 arranged within the collection chamber 10, inside said hollow head 2. This saliva absorption means 20, in use, absorb the saliva flowing into said hollow head 2 in a more efficient manner, because the saliva absorption means 20 draw the saliva from the lower part of the user's mouth into the collection chamber 10. This drawing effect is especially efficient and allows accumulating even more saliva inside the collection chamber 10, than with the hollow head 2 alone.

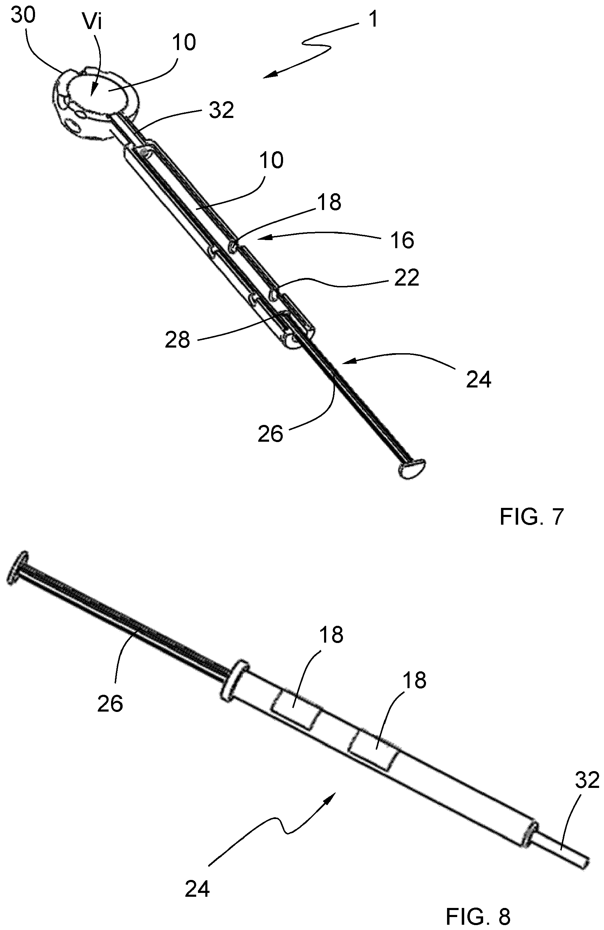

On the other hand, the arrangement of saliva absorption means 20 reduces the amount of saliva naturally flowing down to the sensing means 16. To solve this problem, it is preferable that the hollow handhold means 4 further comprises saliva displacement means 24 fluidly associated to the saliva collection chamber 10 to cause saliva collected during the use of the device 1 to be drawn from the hollow head 2 towards the sensing films 18, located near to the second end 8 of the hollow handhold means (see figures 7 and 8).

The preferred saliva displacement means 24 of this embodiment comprises an inner plunger 26 with a plunger head 28. It is especially preferable that the plunger 26 is part of a syringe inserted in the hollow handhold means 4 of the device 1. However, it cannot be discarded that the inner plunger is guided directly inside the inner wall of the hollow handhold means 4.

Figure 7 shows how the inner plunger 26 is slidably mounted between an inserted position (not shown) in which the plunger head 28 is adjacent to the hollow head 2 and a remote position (see Figure 7) in which the plunger head 28 is located near the second end 8. To efficiently collect the saliva sample, in this remote position, the plunger head 28, is located beyond the sensing means 16, to the opposite to the side of said first end 6. This achieves that all the saliva sample is drawn down towards the sensing films 18. The plunger head 28 may comprise a rubber seal at the top to ensure a better vacuum inside the hollow chamber 4.

Additionally, the syringe of this embodiment comprises a tubular tip 32 at the first end 6 having a smaller cross section than the cross section of the hollow handhold means 4. The tubular tip 32 reaches the inner volume Vi of the hollow head 2 and is fluidically connected to said saliva absorption means 20 to efficiently draw the saliva accumulated in the absorption means towards the sensing films 18 located at the bottom of the syringe.

The syringe also comprises through holes as a part of the optical path 14 for the detection of the analyte to be determined from the saliva sample.

The position of these holes in the cylinder of the saliva displacement means is concentric with respect to the holes in the hollow handhold means 4. These holes are now covered with the sensing films 18 on the outer surface of the syringe. These holes ensure the contact between the saliva and the sensing films 18. Figure 8 shows the suction means 24 of the device 1. The cylinder of the syringe has an overall length of between 50 mm and 100 mm, an outer diameter of between 3 mm and 9 mm and an inner diameter of between 1 mm and 7 mm.

The inner plunger 26 has a length of between 45 mm and 95 mm and a diameter of between 0.8 mm and 6.8 mm. This inner plunger 26 is concentric with the hollow handhold means 4. The rubber seal at the top of the inner plunger 26 has an outer diameter of between 1 mm and 7 mm, an inner diameter of between 0.8 mm and 6.8 mm and a length of between 3 mm and 6 mm.

The saliva displacement means 24 can be made of any transparent material to be compatible with optical measurements, e.g., Smartfil GLACE or Clear GLASSBEND 3D Printer Filament, among many others. Since the material does not come into contact with the user's mouth, it does not need to meet specific biocompatibility specifications. In any case, any polymeric material which is transparent or translucent and compatible with optical measurements can be suitable in the context of the invention.

Also in this embodiment, further to the saliva enhancing shape of the device 1, the hollow head 2 further comprises an outer saliva secretion enhancing layer 30.

The saliva secretion enhancing layer 30 is inert, that is, it is an edible material such as gelatine, caramel or any other material that performs the same function. However, it does not contain any components that may alter the detection of the analyte of interest in the saliva sample. This saliva secretion enhancing layer 30 must be pleasant for the user. The saliva secretion is preferably enhanced using acidic flavours.

This saliva secretion enhancing layer 30 is especially beneficial for paediatric users or patients with problems to produce saliva by other methods due to poor salivation (people with diabetes, people who have suffered a stroke, people with oral candidiasis, people with Alzheimer's disease, or people with autoimmune diseases such as Sjogren's syndrome or HIV and AIDS, among others). The saliva secretion enhancing layer 30 is based on natural, non-sweetened materials, such as Xylitol, to provide different flavours and sweeten without the need for sugar.

This kind of saliva enhancing layers 30 do not cause caries due to their non-sugar content, and can also be used for patients with obesity, type 2 diabetes or with oral problems or treatments that discourage the consumption of sugar. The device is also suitable for allergic users, e.g., celiac patients or the like.

Once the saliva has been properly collected and the sensing films 18 are well wet with the saliva sample, the saliva can be analysed to diagnose the patient's disease.

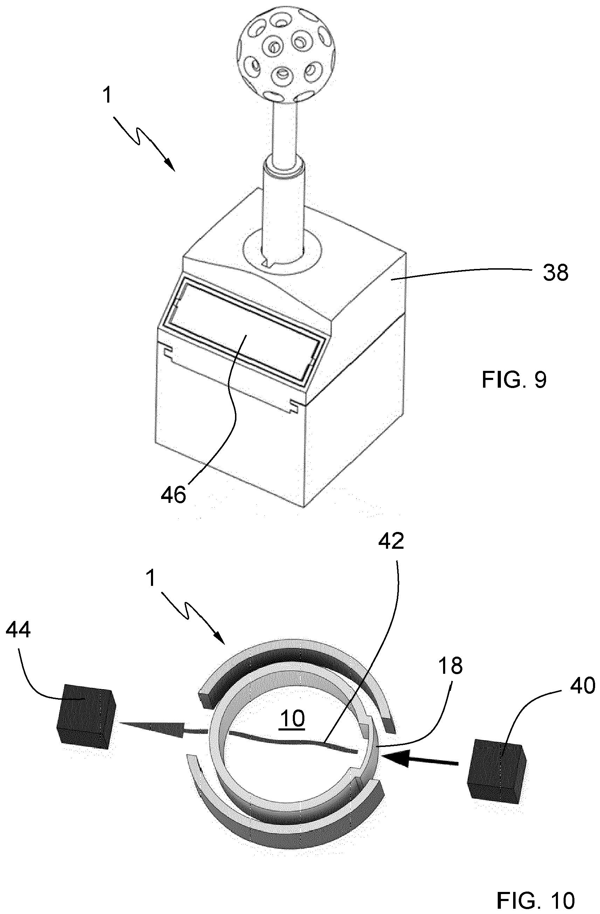

As it is apparent from Figure 9 in this embodiment the saliva sensing means 6 further comprises a housing 38 containing the optical electronics to carry out the diagnosis of the patient's disease of the user's target analyte.

To this end, the sensing means 6 include a light emitting source 40 such as an LED RGB arranged to illuminate the sensing films 18 with a light beam 42, a light receiving sensor 44, such as a photoresistor, a camera or the like, arranged facing the light emitting source 40 but at the opposite side of the sensing films 18 to receive the light beam 42 crossing through the corresponding sensing film 18, and processing means not shown in detail in the figures, but contained within the housing 38 , like an electronic microcontroller board (e.g. an Arduino Nano^{®}) functionally associated to the light emitting source 40 and the light receiving sensor 44 to analyse the light beam 42 and to correlate it with the disease to be diagnosed.

More particularly to simplify the design the light emitting source 40 and light receiving sensor 44 are connected to the processing means and are arranged inside the optical device in such a way that they face each other on both sides of the light path 14 and coincide with the holes 22. The saliva sample does not come into contact with the person handling the device 1 at any time, nor with the electronic components of the device 1, and cannot contaminate the sample or damage the electronics under any circumstances. Thus, the optical device can be employed multiple times.

The device allows measurements to be taken of the saliva sample at specific absorbance values, which are coincident with the wavelength of the peaks of the absorbance spectrum associated with the analyte to be determined. The device 1 makes it possible to obtain a voltage value measured by the light receiving sensor, which will be lower the less light reaches it from light emitting source 40. In other words, it will be lower, the more light is absorbed by the sensing film 18 containing the saliva sample (at the wavelength emitted by the light emitting source 40).

To facilitate the interpretation of the result by the user, device 1 further comprises a software which is designed such that the signal received from the light receiving sensor 44 is translated into a concentration value of the analyte to be assessed, which is displayed in the corresponding display 46 of the device 1.

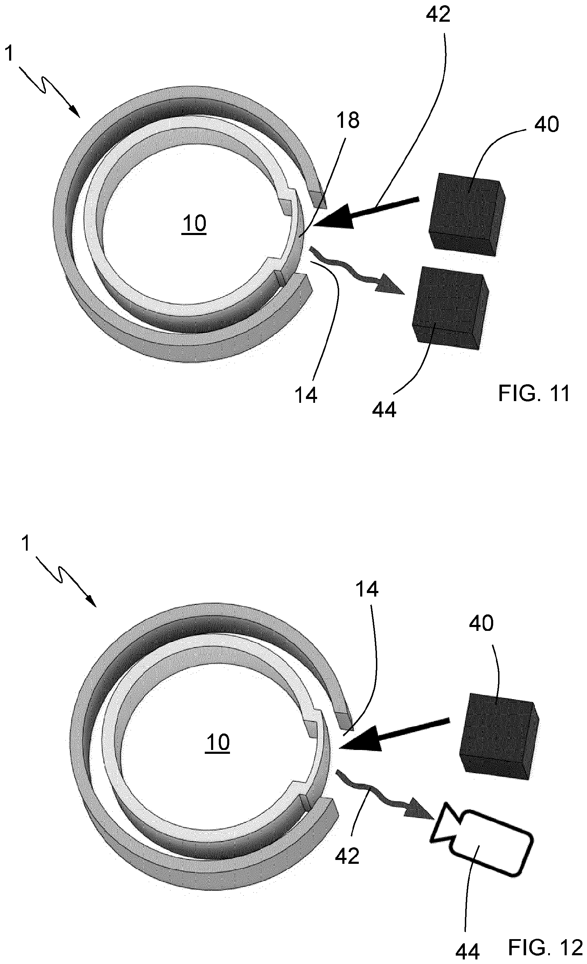

Figure 11 shows a partial cross-section view in perspective of a second alternative embodiment of the optical path 14 of the device according to the invention. In this case and differently to the embodiment of Figure 19, the light receiving sensor 44 is arranged facing said the sensing film 18, but on the same side of the sensing film as the side where the light emitting source 40 is arranged. Therefore, the light receiving sensor 44 receives light beam 42 reflected by the sensing film 18.

Finally, in the embodiment of optical path 14 of Figure 12 to increase the precision of the analyte measurement, the light receiving sensor 44, instead of it being a photoresistor, in this case it is a camera also functionally connected to processing means to carry out the corresponding sample analysis. Also, the camera is facing the sensing film 18 at the same side as the light emitting source 40.

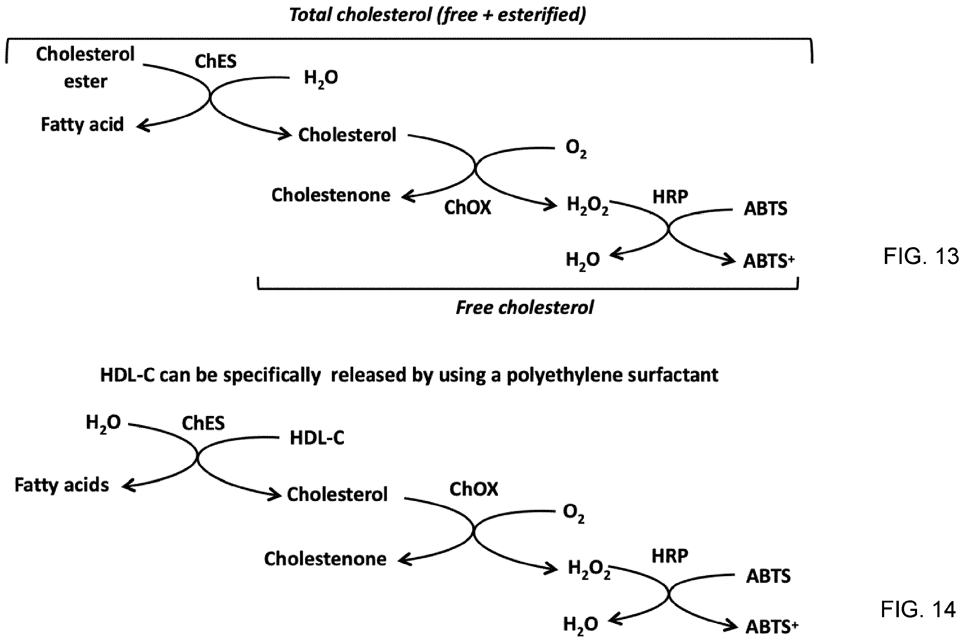

An example of application of the device 1 according to the invention is described below with reference to Figures 13 to 16.

The saliva monitoring oral device 1 of this preferable non-limiting embodiment, has been adapted for cholesterol determination in a paediatric population. In this case, it is important to determine both total cholesterol and non-HDL cholesterol (non-High-density lipoprotein).

For this purpose, two different types of enzymatic sensors are integrated into the sensing films 18. In the case of total cholesterol (see Figure 13), the enzymatic process is as follows: the ester cholesterol in the saliva sample (about 70% of the total) is released in the presence of the ChES enzyme (cholinesterase) and remains all in the form of free cholesterol. Total cholesterol reacts with oxygen in the presence of the enzyme cholesterol oxidase, producing hydrogen peroxide. Hydrogen peroxide reacts with the mediator ABTS (2,2'-azino-bis(3-ethylbenzothiazoline-6-sulfonic acid) in the presence of the enzyme HRP (horseradish peroxidase), oxidising ABTS to its ABTS+ form. The concentration of oxidised ABTS is directly proportional to the concentration of total cholesterol in the saliva sample. This oxidised form of ABTS is coloured (please note that neutral ABTS is colourless), and this colour change allows to determine the cholesterol concentration.

The second sensor allows the determination of HDL cholesterol in the saliva sample. In this case, the enzymatic reaction is the same, but with the addition of a step consisting of adding a surfactant from the polyethylene family to selectively release HDL cholesterol from the saliva sample. After that, the cascade reaction is the same: the HDL ester is released due to the presence of the ChES enzyme. The resulting total HDL in the presence of ChOX (Cholesterol oxidase) reacts with oxygen to hydrogen peroxide. Peroxide in the presence of the HRP enzyme reacts with ABTS, oxidising it to ABTS+. The oxidised ABTS is proportional in this case to the HDL cholesterol in the saliva sample. Non-HDL cholesterol is determined as the difference between total cholesterol and HDL cholesterol.

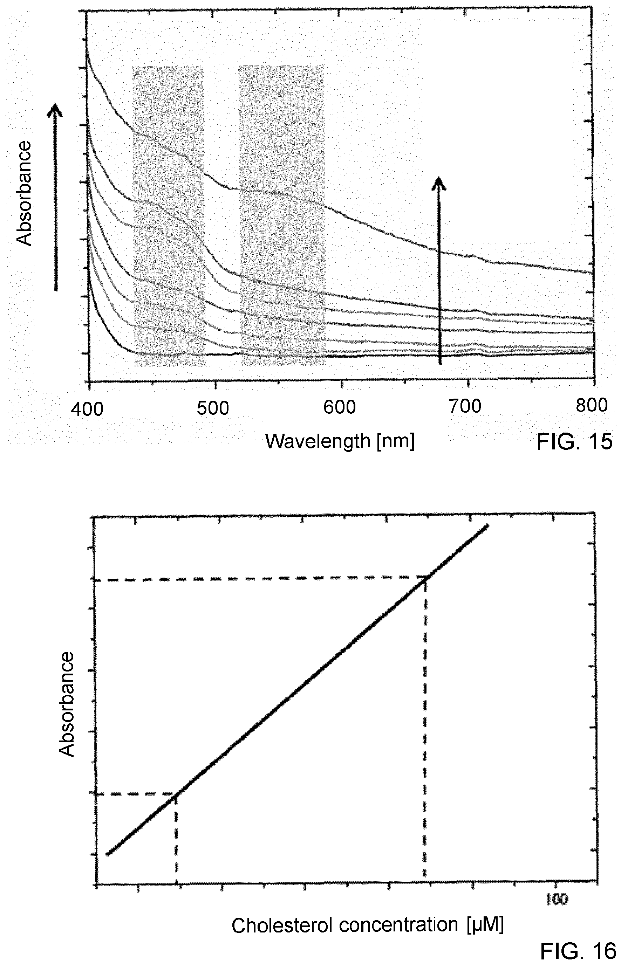

The optical absorption spectrum resulting from these reactions shows two absorption peaks related to the concentration of the analyte of interest (cholesterol according to the example reactions in Figure 15) at wavelengths of approximately 430 - 470 nm and 530 - 570 nm. These wavelength values correspond to the absorption of the green colour of the ABTS+ and the purple colour resulting from the interaction between the ABTS+ and the biosensor matrix that immobilises the reagents on the surface of the sensing films 18 device (Figure 16). As the concentration of the analyte of interest in the collected saliva sample increases, the resulting concentration of oxidised ABTS will be higher and higher, resulting in higher value absorbance spectra. The resulting absorbance values of the different saliva samples are interpolated to a previously constructed calibration line for the analyte of interest (adapted to the range of interest of the analyte to be analysed), resulting in the exact value of the concentration of the analyte in the sample can be displayed in the display 46 of the analysis device of Figure 9.

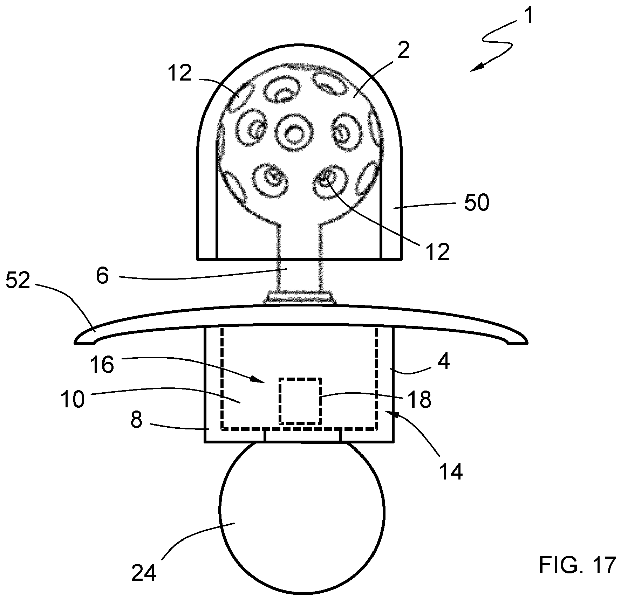

Finally, Figure 17 shows a third embodiment of the saliva monitoring oral device 1 for collecting and monitoring a saliva sample according to the invention.

Again, the device 1 comprises a head 2 configured to be received in the user's mouth, and longitudinal handhold means 4 with a first and a second ends 6, 8.

However, in this case, the device 1 is not shaped as a lollipop, but as a pacifier. Therefore, the handhold means 4 is also a hollow cylindrical container, which is much shorter than the hollow shaft of the previous embodiments.

However, the head 2 and said handhold means 4 are both hollow and fluidically connected to one another at said first end 6 of the hollow handhold means 4 to define the saliva collection chamber 10.

The hollow head 2 is further rigid, insoluble in saliva and comprises a plurality of inlet through holes 12. The plurality of inlet through holes 12 are fluidically connected to the saliva collection chamber 10.

This device morphology is especially adapted for little children, e.g., under 5 years and again profits from the suction reflex caused by the device 1 to enhance the saliva secretion. In use, the saliva contained from the user's mouth easily enters the saliva collection chamber 10.

At the second end 8 the device 1 further comprises saliva sensing means 16 comprising a reactive agent for characterising said saliva sample as explained in previous embodiments. Furthermore, the device comprises a shield 52 arranged between the first and the second ends 6, 8, avoiding that the using infant has any oral contact with the reactive agent during use, thus increasing the security of use of the device.

The handhold means 4 is made of a transparent plastic material, such than the sensing film 18 is arranged to cover the optical path 14, which in this case is the own wall of the handhold means 4.

The device 1 further comprises saliva displacement means 24 which are a deformable elastomeric suction head 48 fluidly associated to the saliva collection chamber 10. Upon depressing the suction head 48, the saliva collected during the use is displaced from the absorption means of the hollow head 2 (no shown in detail) towards the sensing film 18 for later analysis.

Alternatively, this embodiment could not have the absorption means inside the hollow head 2 and would thus not need the saliva displacement means 24, as it was the case of the first embodiment.

Finally, this embodiment further comprises a protecting cap 50 intended to close, preferably in a sealing manner, the hollow head after the saliva sample has been collected. Thanks to this protecting cap 50, the saliva sample can be transported in a secure and hygienic manner to a laboratory for detailed saliva characterization at a later moment. The protecting cap 50 could also be used in any other embodiment described in the previous paragraphs.

As already mentioned, the device according to the invention allows collecting larger amounts of saliva than the devices of the prior art and can be applied to the collection and monitoring a saliva sample of a user to characterise the saliva such as disease diagnosis, drug testing, or the like.

## Claims

1. A saliva monitoring oral device (1) for collecting and monitoring a saliva sample of a user comprising:
[a] a head (2) configured to be received in the user's mouth, and
[b] longitudinal handhold means (4) with a first and a second ends (6, 8), **characterised in that**
[c] said head (2) and said handhold means (4) are both hollow and fluidically connected to one another at said first end (6) of said hollow handhold means (4) to define a saliva collection chamber (10),
[d] said hollow head (2) is rigid, insoluble in saliva and comprises a plurality of inlet through holes (12), said plurality of inlet through holes (12) being fluidically connected to said saliva collection chamber (10) for allowing, in use, saliva from the user's mouth to enter said saliva collection chamber (10),
[e] said device (1) further comprises saliva sensing means (16) comprising a reactive agent for characterising said saliva sample.

2. The saliva monitoring oral device (1) according to claim 1, **characterised in that** said saliva sensing means (16) further comprises at least one sensing film (18) and said reactive agent is contained in said at least one sensing film (18), said at least one sensing film (18) being arranged inside said hollow handhold means (4), in fluidical communication with said saliva collection chamber (10), distanced from said hollow head (2), such that, in use, said at least one sensing film (18) is impregnated by the saliva collected inside said saliva collection chamber (10).

3. The saliva monitoring oral device (1) according to claims 1 or 2, **characterised in that** said hollow handhold means (4) comprises at least one optical path (14) located between said first and second ends (6, 8) and distanced from said hollow head (2), and **in that** said at least one sensing film (18) is arranged to cover said optical path (14).

4. The saliva monitoring oral device (1) according to any of claims 1 to 3, **characterised in that** said head (2) has an inner volume of at least 2 mL.

5. The saliva monitoring oral device (1) according to any of claims 2 to 4, **characterised in that** it further comprises saliva absorption means (20), said saliva absorption means (20) being arranged within said collection chamber (10), inside said hollow head (2), to absorb, in use, saliva flowing into said hollow head (2).

6. The saliva monitoring oral device (1) according to any of claims 3 to 5, **characterised in that** said optical path (14) comprises at least one monitoring through hole (22) on said hollow handhold means (4).

7. The saliva monitoring oral device (1) according to claim 6, **characterised in that** said at least one monitoring through hole (22) extends through the cross section of said hollow handhold means (4).

8. The saliva monitoring oral device (1) according to any of claims 1 to 7, **characterised in that** said hollow head (2) has an overall size of at least 15 mm.

9. The saliva monitoring oral device (1) according to any of claims 2 to 8, **characterised in that** said device (1) further comprises saliva displacement means (24) fluidly associated to said saliva collection chamber (10) to cause saliva collected during the use of said device to be displaced from said hollow head (2) towards said sensing film (18).

10. The saliva monitoring oral device (1) according to claim 9, **characterised in that** said saliva displacement means (24) comprises an inner plunger (26) with a plunger head (28), said inner plunger (26) being, slidably mounted between
[i] an inserted position in which said plunger head (28) is adjacent to said hollow head (2) and
[ii] a remote position in which said plunger head (28) is located near said second end (8), beyond said sensing means (16), to the opposite to the side of said first end (6).

11. The saliva monitoring oral device (1) according claim 10, **characterised in that** said hollow handhold means (4) comprises a tubular tip (32) at said first end (6) having a smaller cross section than the cross section of the hollow handhold means (4) and **in that** said tubular tip (32) reaches the inner volume (Vi) of said hollow head (2) and is fluidically connected to said saliva absorption means (20).

12. The saliva monitoring oral device (1) according to any of claims 1 to 11, **characterised in that** said hollow head (2) further comprises an outer saliva secretion enhancing layer (30), said saliva secretion enhancing layer (30) being chemically inert both for the user and for the saliva sample.

13. The saliva monitoring oral device (1) according to any of claims 1 to 12, **characterised in that** said sensing film (18) is distanced a distance (L) of at least 20 mm from the outer surface (S) of said hollow head (2) and preferably at least 30 mm.

14. The saliva monitoring oral device (1) according to any of claims 1 to 13, **characterised in that** said saliva sensing means (16) comprises at least two sensing films (18), separated from each other in the longitudinal direction of said hollow handhold means (4) and each sensing film (18) contains different reactive agents.

15. The saliva monitoring oral device (1) according to any of claims 2 to 14, **characterised in that** said saliva sensing means (6) further comprises:
[a] a light emitting source (40) arranged to illuminate said at least one sensing film (18) with a light beam (42),
[b] a light receiving sensor (44), arranged facing said at least one sensing film (18) to receive said light beam (42) from said at least one sensing film (18), and
[c] processing means functionally associated to said light emitting source (40) and said light receiving sensor (44) to analyse said light beam (42) and to correlate it with the disease to be diagnosed.
